# EUROPEAN PATENT APPLICATION

(11) **EP 3 097 892 A1**
(43) Date of publication of application: **30.11.2016**
(21) Application number: 15169828.9
(22) Date of filing: 29.05.2015
(51) Int. Cl.: A61F 9/00, A61F 9/007

(54) **CANNULA AND INSTRUMENT FOR INSERTING A CATHETER**

(71) Applicant: Klinikum rechts der Isar der Technischen Universität München, 81675 München (DE)
(72) Inventor: Nasseri, Mohammad Ali, 80807 München (DE); Maier, Mathias, 81667 München (DE); Lohmann, Chris Patrick, 81545 München (DE)
(74) Representative: Lucke, Andreas

(57) **Abstract**

A cannula for inserting a catheter is provided. The cannula comprises a first channel (30) for receiving and guiding a catheter tube (26). The first channel (30) extends to a first opening (22) and has an end portion defining an insertion direction (I), such that a catheter tube (26) when being advanced and guided through the end portion of the first channel (30) exits the cannula through the first opening (22) in the insertion direction (I). The cannula further comprises a second channel (32) for receiving a pointing device for providing a pointing beam. The second channel (32) extends to a second opening (24) and has an end portion (34) defining a pointing direction (P). The pointing direction (P) points from the second opening (24) towards a location, which is located outside the cannula along the insertion direction (I) in front of the first opening (22).

## Description

### Technical Field

The present invention relates to cannulation. In particular, the present invention relates to a cannula, an instrument and a method for inserting a catheter.

### Background

Cannulation, i.e. the insertion of a catheter, of a hollow needle or of a cannula in a tissue, is used in medical treatments and surgeries, such as in ophthalmic surgeries. Cannulation can also be used in other non-medical applications where the insertion may not be performed on a tissue but on another ground.

A sub-discipline of ophthalmic surgeries are vitreo-retinal surgeries, where retinal vessels and sub-retinal layers may be cannulated, e.g. for the treatment of vein occlusion diseases, such as a diabetic retinal vein occlusion and for the treatment of Aged-related Macular Degeneration (AMD). For the treatment by use of instrumental micro cannulation liquids may be injected into a tissue and/or extracted from a tissue.

EP 1 738 790 A1 provides an example for a device for the injection of drugs into a retinal micro vessel by using a catheter. The catheter is carried by a sleeve which can be mounted to an endoscope which is used in vitreo-retinal surgery. The catheter has a sharp tip which can be introduced into a retinal vessel under visualization and control with the endoscope.

Another example for an instrument which is suitable for microsurgical injection is provided in US 2003/0171722 A1. The instrument comprises a hollow miniaturized needle for penetrating a retinal vessel. The needle may carry a flexible tube which is movable through the needle. The needle may be illuminated by use of an optical fiber.

### Summary of the Invention

The object of the present invention is to provide a cannula, an instrument and a method which allow for an improved cannulation, in particular for a precise and safe cannulation.

This object is solved by the independent claims. Preferred embodiments are defined in the dependent claims.

The present invention relates to a cannula for inserting a catheter, wherein the cannula comprises a first channel for receiving and guiding the catheter. The first channel extends to a first opening and has an end portion defining an insertion direction, such that the catheter when being advanced and guided through the end portion of the first channel exits the cannula through the first opening in the insertion direction. The cannula further comprises a second channel for receiving a pointing device for providing a pointing beam. The second channel extends to a second opening and has an end portion defining a pointing direction. The pointing direction points from the second opening towards a location, which is located outside the cannula along the insertion direction in front of the first opening.

The cannula can be used in combination with a suitable pointing device and a suitable catheter. A suitable pointing device can be received in the second channel and can provide a pointing beam in the pointing direction when received in the second channel. A suitable catheter can be advanced through the cannula to exit the first opening in the insertion direction. The cannula is formed, such that the pointing direction points from the second opening towards a location, which is located outside the cannula along the insertion direction in front of the first opening. Accordingly, there is a location which is defined by the cannula and which corresponds to an intersection of the pointing direction and the insertion direction. The pointing beam of a pointing device along the pointing direction can be used to indicate or mark the location of the intersection with respect to a target location on a ground, e.g. a tissue or a vessel. Because the location of the intersection can be marked, it is possible to adjust the position and the orientation of the cannula, such that the intersection coincides precisely with the target location. In this position and orientation of the cannula - where the intersection and the target location coincide - the insertion direction of the cannula intersects the target location, such that the catheter can be advanced through the cannula to penetrate the ground at the target location. Therein, the insertion can be performed just by advancing the catheter through the cannula without need to move the cannula relative to the ground. Further, during the insertion or penetration the position and orientation of the cannula can be fixed and stabilized while the pointing beam may still be used to verify the correctness of the position and orientation of the cannula. In other words, since the cannula allows for separating the step of positioning from the step of inserting, it can be ensured that a penetration of the ground only takes place when the cannula is correctly positioned and orientated and may be stabilized in its position. The insertion itself can be performed just by advancing or moving the catheter trough the cannula. Hence, the cannula allows for an easy and precise adjustment by monitoring and controlling the position and orientation of the cannula and for a precise and safe insertion of a catheter at a target location.

In the cannula, the second channel may extend parallel to the first channel and/or the first channel may protrude a leading end of the second channel. If the channels are parallel, the cannula can have a smaller diameter which is advantageous when using the cannula for surgeries where the cannula may be introduced in a tissue. If the first channel protrudes a leading end of the second channel, the pointing beam may propagate from the second opening in a direction which is adjacent and parallel to a protruding portion of the first channel. Accordingly, the space which is required for the cannula, for the pointing device and for the propagation of the beam may have a longitudinal shape with a small diameter which is advantageous for applications with corresponding spacial requirements, such as ophthalmic surgeries.

The cannula may have an end portion located at the first opening which is bent. Accordingly, the insertion direction can be defined by a bent end portion of the first channel extending in the bent end portion of the cannula. Therefore, the intersection of the pointing direction and the insertion direction may be provided only by the path of the channel end portions while the first and second channels may extend essentially parallel outside the end portions. This allows for a thin longitudinal device and for keeping the lateral extension of the cannula small.

Preferably, the first channel and the second channel are formed in the same single part of material. This allows for a compact arrangement without having a connection between different parts in which the individual channels would be formed, respectively. This allows for a cannula having a high stability and for a cannula with a smooth and even outer surface without interruptions or irregularities.

The cannula may be a blunt cannula. Since the insertion or penetration does not need to be performed with the cannula but can be performed with a catheter, the cannula does not need to be pointed or sharpened. In medical applications a blunt cannula may reduce the risk of creating unwanted violations or penetrations in the tissue, compared to a cannula having a pointed tip.

According to one or more preferred embodiments, the cannula further comprises a support element being attached to an end portion of the cannula for stabilizing the cannula when the cannula is forced, e.g. along its length direction, against a ground, wherein the supporting element preferably comprises a material which is softer than the material in which the first and second channels are defined.

The support element may allow stabilizing the position and orientation of the cannula after the cannula has been positioned and orientated for the insertion of a catheter and when the cannula touches a ground with the support element close to the target location. The support element may also allow for pinching off a vessel thereby leading to a swelling of the vessel which alleviates the vessel cannulation by the catheter. Preferably, the support element is arranged at the position of the cannula, in which it touches the ground and supports and/or stabilizes the cannula, when the cannula is correctly positioned and orientated, i.e. when the intersection of the pointing direction and the insertion direction coincides with the target location, and when the cannula is pressed or forced against the ground. With respect to the surface of the ground, the correct position and orientation of the cannula may be an upright or angled position or orientation, for example.

If the support element comprises a soft material, the friction between the cannula and the ground, e.g. a tissue can be increased and a slippage of the cannula during the insertion can be avoided. Further, by using a soft material the risk of violating a tissue can be reduced.

According to one or more embodiments of the cannula, the first channel may comprise a portion having a first diameter which is larger than a second diameter of the end portion of the first channel, wherein the first diameter is preferably between 70 µm and 200 µm, in particular about 100 µm, and/or wherein the second diameter is preferably between 30 µm and 80 µm, in particular about 50 µm.

The portion of the first channel having a larger diameter can be used to receive a catheter portion having a correspondingly large diameter which may serve as a fluid tank or fluid reservoir. This is useful when using the cannula to inject a fluid into a tissue and/or to remove a fluid from a tissue. The thinner portion of the first channel allows receiving and guiding a portion of a catheter which may be correspondingly thin. This may be advantageous for micro cannulation when small tissue structures, such as micro vessels, are penetrated.

In a preferred embodiment, which can be used for ophthalmic surgeries, the cannula has a length of between 30 mm and 60 mm, preferably of about 40 mm, and/or a rear portion having a diameter of between 400 µm and 700 µm, preferably of about 560 µm, and/or a middle portion having a diameter of between 100 µm and 400 µm, preferably of about 200 µm, and/or an end portion having a diameter of between 60 µm and 150 µm, preferably of about 80 µm. For other applications, which also include non-medical applications, the cannula can have different dimensions.

The present invention further relates to an instrument comprising a cannula according to one or more of the above-mentioned embodiments and a pointing device which is received in the second channel of the cannula.

The present invention further relates to an instrument for inserting a catheter, which - different from the aforementioned embodiments - comprises a cannula which may not have a second channel for receiving a pointing device. This instrument comprises a cannula having a channel corresponding to the first channel which has been described above and which defines an insertion direction. The instrument further comprises a pointing device for providing a pointing beam in a pointing direction, wherein the pointing device is attached to the cannula and relatively arranged to the cannula, such that - when a pointing beam is provided - the pointing beam points and propagates towards a location, which is located outside the cannula along the insertion direction in front of an opening corresponding to the first opening which has been described above. Hence, in this embodiment, the pointing direction is not defined by a second channel but is defined by a pointing device being attached to the cannula. It is not necessary that the pointing device is in direct contact with the cannula. However, the pointing device is arranged with respect to the cannula, such that it is configured to define a pointing direction with respect to the position and orientation of the cannula. For example, the pointing device and the cannula may be rigidly connected, such that the movement of the cannula causes a corresponding simultaneous movement of the pointing device.

The pointing device of some or all of the aforementioned embodiments may comprise an optical fiber and/or a lens. The optical fiber can be received in the second channel, for example, or may be attached to the cannula. In some examples, the fiber can be part of or may correspond to an Optical Coherence Tomography (OCT) probe.

The lens can be arranged at the second opening or at another position at an end of the optical fiber, for example. The lens allows for providing a collimated or focused beam, which - different from a divergent illumination - is suitable for optically marking a target location on a ground by an optical spot or light spot. The spot may be magnified by using corresponding optics and may be directly visible, if light in the visible range is used, or may be visualized, if wavelengths are used, which are not directly visible.

The pointing device may further comprise a light source, such as a laser, a laser diode or an LED.

In one or more of the aforementioned embodiments, the instrument may further comprise a handle portion, wherein the handle portion preferably comprises a mechanism for moving a catheter and/or a mechanism for fluid injection and/or fluid removal. The mechanism for moving a catheter may, for example, comprise a piezo motor. The mechanism for fluid injection and/or fluid removal may comprise, for example, a piston which can be pushed into the catheter or drawn back within the catheter. The movement of the piston may be performed manually and/or electronically by a corresponding device, such as a robot.

Further, the handle portion and the cannula may be detachably connectable. This allows using a disposable cannula which may only be used for a single application, e.g. a surgery, while the handle portion may be used for a number of applications. This is particularly useful for applications which may require an uncontaminated and sterilized cannula or for applications where it is likely that the cannula is damaged or destructed.

The present invention further relates to an instrument comprising a cannula according to one or more of the before mentioned embodiments and further comprising a corresponding catheter which can be received and guided within the cannula.

The catheter preferably has a leading end which is pointed. This allows for a better penetration of a ground, such as the penetration of a micro vessel. According to a preferred embodiment, the catheter may comprise a catheter tube and a preferably flexible mandril which can be received and guided in the catheter tube and which can have a leading end which is pointed.

Further, the catheter may have a portion with a larger diameter compared to a leading portion of the catheter. The larger portion can be used as a fluid tank or fluid reservoir for fluids to be injected into a tissue and/or removed from a tissue.

Further, the provision of a portion with a larger diameter and a portion with a smaller diameter may lead to a shoulder portion or taper portion of the catheter where the diameter reduces abruptly or over a certain length to a smaller diameter. This portion may serve as a stopper, such that the catheter may only be advanced to a specific and defined end position where the stopper abuts against a portion of the cannula and avoids a further advancing. The position of the stopper along the catheter and the length and shape of the corresponding channel in the cannula may define a maximum length by which the catheter can be pushed or moved out of the cannula.

A possible application of the instrument of one or more of the aforementioned embodiments is the ophthalmic surgery, such that the instrument may be configured to provide the pointing beam inside an eye ball, preferably inside a human eye ball.

However, the present invention also comprises embodiments which may not be suitable for ophthalmic surgery. This might be due to unsuitable dimensions or proportions of the cannula, e.g. when the origin of the pointing beam is too remote from the opening where the catheter exits the cannula, or might be due to an unsuitable wavelength or intensity of the pointing beam. In other applications such dimensions, proportions or wavelengths can be suitable.

The present invention further relates to a method for inserting a catheter, preferably into a blood vessel, wherein the method comprises
providing a pointing beam with a pointing device, wherein the pointing beam has a defined path relative to a cannula and intersects an insertion direction defined by the cannula,
positioning the cannula relative to a target location on a ground, e.g. a tissue, such that the cannula contacts the ground and such that a location of a spot created by the pointing beam on the ground is located at the target location,
advancing the catheter trough the cannula, such that the catheter exits the cannula in the insertion direction and penetrates the ground at the target location.

### Brief Description of the Drawings

Further advantages and details of the present invention will be understood from the following description in which preferred embodiments are described with reference to drawings. In the drawings same reference numerals designate corresponding features.
- Fig. 1: shows an instrument comprising a cannula according to an embodiment of the present invention.
- Fig. 2: shows a portion of the cannula illustrated in Fig. 1.
- Fig. 3: shows a portion of the cannula portion illustrated in Fig. 2.
- Fig. 4: shows a cannula portion of the cannula portion illustrated in Fig. 2.
- Fig. 5: shows a top view on a longitudinal section through the cannula portion illustrated in Fig. 2.
- Fig. 6: shows a cross-section of the cannula portion illustrated in Fig. 2.
- Fig. 7: illustrates an application of the instrument of Fig. 1 which is inserted into an eye ball.
- Fig. 8: shows a cross-section through the eye ball of Fig. 7 in which the cannula of Fig. 1 is inserted.
- Fig. 9: is a magnification of a portion illustrated in Fig. 8.
- Figs. 10 a), b): illustrate a portion of the cannula of Fig. 1 which is positioned on a blood vessel.

### Detailed Description of the Drawings

In the following description the present invention will be described in respect to a medical application in which the ground in which a catheter may be inserted corresponds to a tissue. However, this is only a specific example of an application. The present invention is not restricted to medical applications but also relates to other applications.

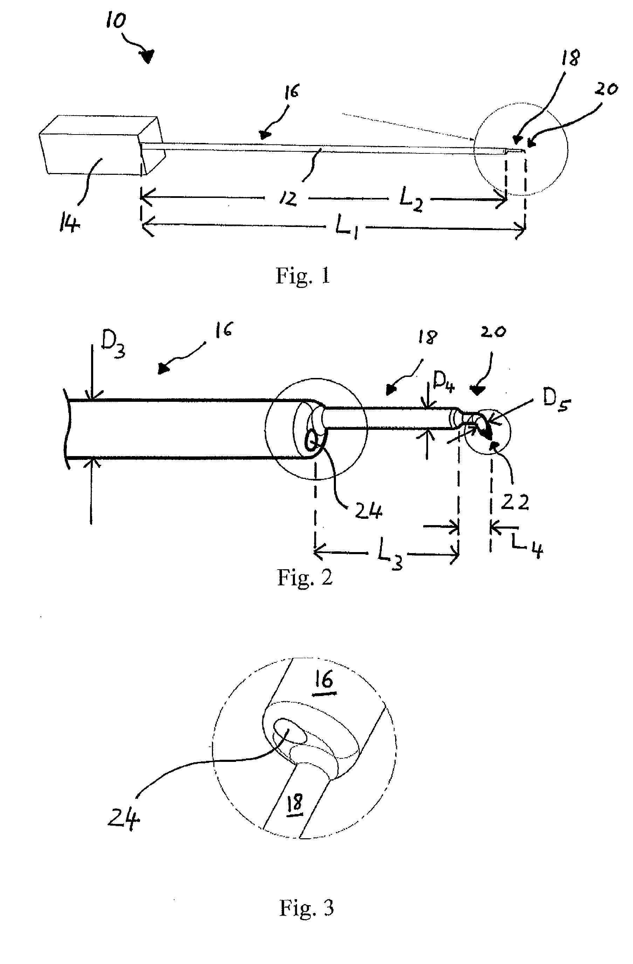

Fig. 1 illustrates an instrument 10 according to an embodiment of the present invention. The instrument 10 comprises a cannula 12 and a handle portion 14. A portion of the cannula 12 which is located at a leading end of the cannula 12 and which is enclosed in the circle of Fig. 1 which is indicated by an arrow is illustrated in Fig. 2. As can be seen in Fig. 2, the cannula 12 may comprise a rear portion 16, a middle portion 18 and an end portion 20, wherein the rear portion 16 may have a larger diameter than the middle portion 18 and the middle portion 18 may have a larger diameter than the end portion 20. As is illustrated in Fig. 1, the middle portion 18 may be arranged between the rear portion 16 and the end portion 20 but must not necessarily be arranged in the middle of the cannula 12.

As is illustrated in Fig. 2, the cannula 12 comprises a first opening 22 and a second opening 24 to which corresponding channels (not shown in Fig. 2) extend, wherein the channels are arranged inside the cannula 12. Fig. 3 provides a magnified illustration of the portion of the cannula 12 which is enclosed by the left circle of Fig. 2.

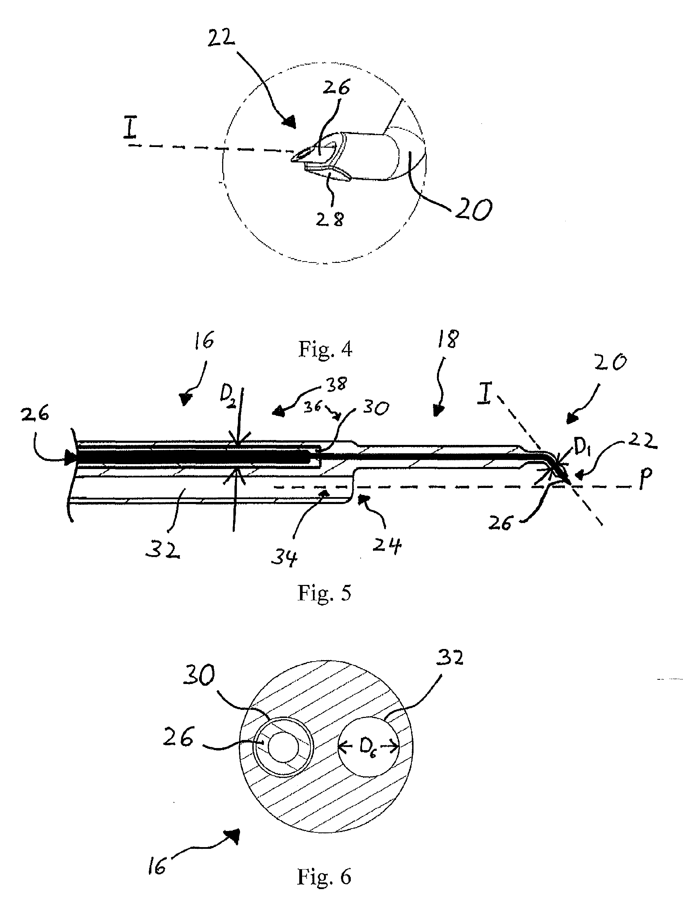

Fig. 4 is a magnified illustration of the inner area of the right circle in Fig. 2 which includes a portion of the end portion 20 of the cannula 12. As is illustrated in Fig. 4, the end portion 20 of the cannula 12 may be bent and may receive and guide a catheter 26 in a corresponding channel (not shown in Fig. 4). The end portion 20 of the cannula 12 or the channel which is defined therein defines an insertion direction I which is indicated by a dashed line in Fig. 4. The insertion direction I corresponds to the direction in which the catheter 26 which is guided and advanced through the end portion 20 of the cannula 12 exits the cannula 12 through the opening 22.

Fig. 4 further shows a support element 28 which is attached to the end portion 20 of the cannula 12 and which has the shape of a plate. The support element 28 may serve to support the cannula 12 against a tissue or any other ground to stabilize the position and orientation of the cannula 12 with respect to the tissue or ground. Preferably, the support element 28 is arranged directly below or adjacent to the opening 22, as is illustrated in Fig. 4, such that it is possible to support the cannula 12 close to a penetration location which is located closely behind the opening 22. Preferably the distance of the support element 28 from the opening 22 is less than ten times the diameter of the opening 22, more preferably less than five times the diameter of the opening 22. This allows for a precise and safe cannulation.

Fig. 5 shows a longitudinal section through the cannula portion which is illustrated in Fig. 2. In the embodiment of Fig. 5, the cannula 12 comprises a first channel 30 extending from the first opening 22 into the cannula 12 and a second channel 32 extending from the second opening 24 into the cannula 12. The second channel 32 comprises an end portion 34 which defines a pointing direction P. The pointing direction P and the insertion direction I are illustrated by dashed lines in Fig. 5 and intersect in front of the first opening 22, as indicated in Fig. 5. When the cannula 12 is adjusted and positioned for catheter insertion, the intersection of the insertion direction I and the pointing direction P is located at a target location on a tissue or on another ground where the catheter 26 will penetrate the tissue or the other ground when being advanced through the positioned cannula 12.

In Fig. 5 the intersection of directions I and P is located close to the opening 22, such that it is possible to perform the penetration with the catheter 26 directly behind the opening 22. Preferably the distance of the intersection from the opening 22 is less than ten times the diameter of the opening 22, more preferably less than five times the diameter of the opening 22. Accordingly, there is no long distance, which the catheter 26 has to cover freely and without guidance after it exits the cannula 12 and before it reaches the target location. This allows for a precise and safe cannulation.

The second channel 32 is configured to receive a pointing device (not shown) which may comprise for example an optical fiber and a lens for collimating or for focusing an optical intensity to provide an optical beam which is suitable for pointing purposes. The lens may be arranged directly at the second opening 24 of the second channel 32. The lens may be an individual element which is separated from the optical fiber or can be a portion of the optical fiber itself, for example, when being formed of a portion of the optical fiber, e.g. by a melting technique.

The present invention includes also other embodiments of instruments, which are not illustrated, and which do not require a second channel for defining the pointing direction P. In these embodiments, the pointing direction P is defined by the position and orientation of the pointing device relative to the cannula 12.

Fig. 5 further illustrates that a first channel 30 may have a portion 36 with a smaller diameter and another portion 38 with a larger diameter. In the example of Fig. 5, the portion 36 with a smaller diameter extends through the end portion 20 of the cannula and the middle portion 18 of the cannula 12. The portion 38 with a larger diameter extends within the rear portion 16 of the cannula 12. Accordingly, a cannula 12 having a first channel 30 with the portions 36, 38 is configured for receiving a corresponding catheter 26 also having a portion with a larger diameter which can be received and guided in the channel portion 38 and having a portion with a smaller diameter which can be received and guided in the channel portion 36. The catheter portion with the larger diameter may serve as a fluid tank or fluid reservoir.

In the embodiment of Fig. 5, the catheter portion with the larger diameter is not receivable in the channel portion 36 with the smaller diameter because its diameter is too large. At the transition from the larger diameter to the smaller diameter the catheter 26 comprises a shoulder which may serve as a stopper. If the catheter 26 is advanced through the cannula 12 until the shoulder reaches the end the channel portion 38, a further advancing is not possible. Accordingly, the maximum length by which the catheter 26 of Fig. 5 can protrude out of the cannula 12 through the first opening 22 corresponds to the length by which the catheter portion having a smaller diameter exceeds the length of the channel portion 36. This maximum protrusion length can be adjusted by varying the corresponding lengths of the catheter and/or channel portions.

Fig. 6 shows a cross-section through the rear portion 16 of the cannula 12.

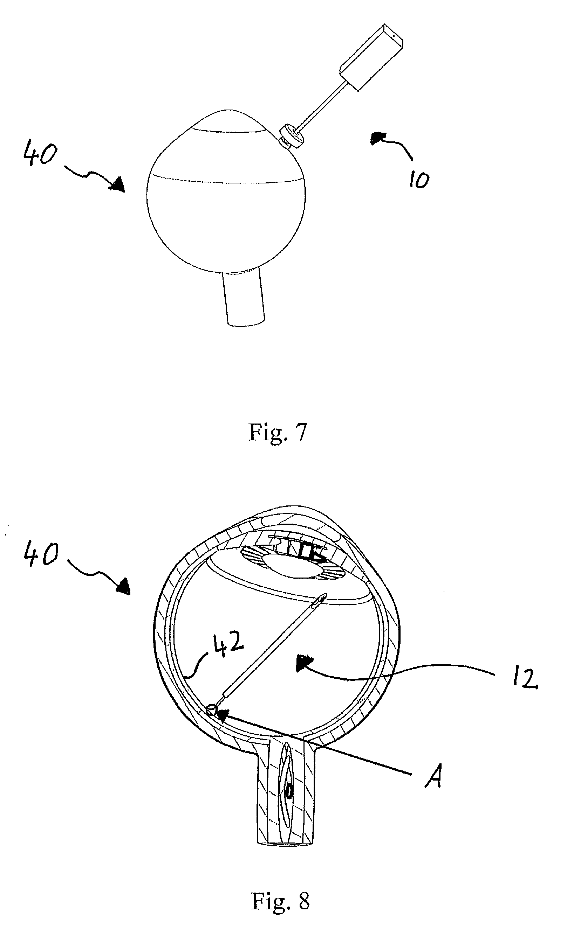

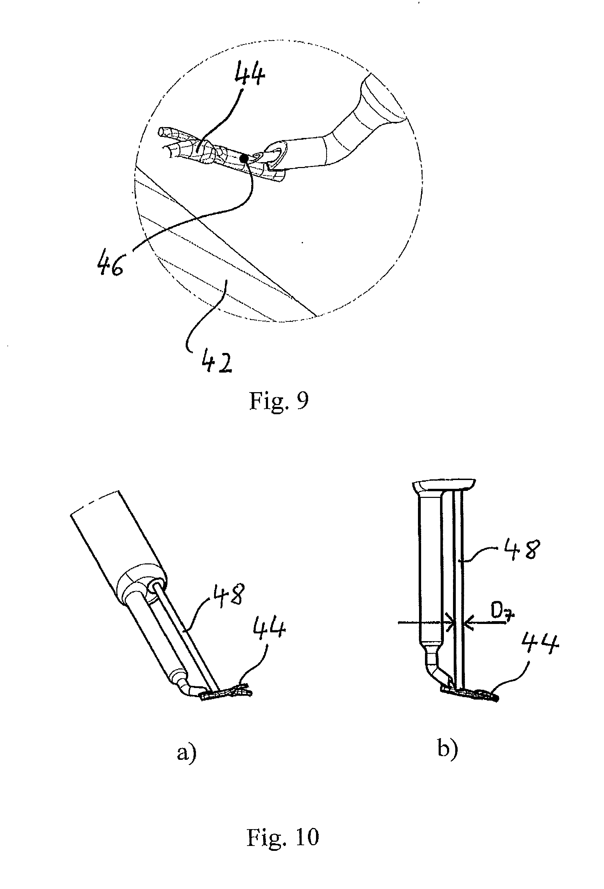

Fig. 7 illustrates an eye ball 40 in which the instrument 10 of Fig. 1 is inserted. Fig. 8 is a sectional view of the eye ball 40 of Fig. 7. Fig. 9 is a magnification of the area inside the circle of Fig. 8 which is indicated by arrow "A". Fig. 8 illustrates the use of the cannula 12 in a vitreo-retinal surgery on the retina 42, in particular at a retinal vessel 44, as is illustrated in Fig. 9. In Figs. 7 to 9 the cannula 12 is positioned with respect to the vessel 44 and stabilized in its insertion position and orientation by the support element 28 which abuts against the vessel 44. The target location 46 where the catheter 26 will penetrate the vessel 44 when it is advanced through the cannula 12 is marked and indicated by a spot which is created by a pointing beam of a pointing device.

Figs. 10a and 10b both illustrate a portion of the illustration of Fig. 8 from different perspectives with an additional illustration of a pointing beam 48 which is not shown in Figs. 8 and 9. In Fig. 9, however, the spot is shown which is created by the pointing beam 48 at the target location 46 on the vessel 44.

For the cannula 12 of the illustrated embodiment the first and second cannels 30, 32 extend essentially in parallel except for the portion of the first channel in the bent end portion 20. Accordingly, the cannula 12, the beam 48 and the intersection of directions I and P can be comprised in a thin longitudinal volume, as illustrated by Figs. 1 and 5. This is advantageous for the application of ophthalmic surgery, where the cannula 12 is inserted in an eye ball 40, as is illustrated in Figs. 7 and 8, and where the lateral extension of the inserted devices needs to be small.

The insertion of a catheter 26 with the cannula 12 can be performed in two separate and sequential steps:

In a first step the cannula 12 is adjusted. In this step the cannula 12 is moved with respect to the retina 42 while simultaneously the spot which is created by the pointing beam 48 on the retina 42 or on the vessel 44 is observed. The cannula 12 can be moved manually by the surgeon or by a device, e.g. by a robot. The movement of the spot indicates a corresponding movement of the cannula 12 and the location of the spot indicates the position of the cannula 12. During observation of the spot, the cannula 12 is moved to bring the spot and the target location 46 on the vessel 44 in coincidence. The adjustment is completed when the intersection of the directions I and P and the target location 46 coincide.

When being adjusted, the cannula 12 according to the embodiment of Fig. 1 touches the vessel 44 with its support element 28 and has a tilted or angled, upright orientation with respect to the vessel 44, as is illustrated in Figs. 10 a) and b). Due to the bent end portion 20 the insertion direction I is only slightly tilted with respect to the vessel 44, when the cannula 12 is adjusted, such that the catheter 26 can penetrate the vessel 44 in the direction of the vessel 44 or with a slight tilt to the direction of the vessel 44. In other embodiments the cannula 12 can have other orientations when being adjusted and must not necessarily touch the vessel 44. In such other embodiments the cannula 12 can have a distance from the ground. Further, when the pointing beam 48 is not collimated, the spot diameter can be used as a measure for the distance from the ground and therefore can also be used for the adjustment. The adjusted cannula 12 can be stabilized in its position and orientation before the catheter 26 is inserted.

In a second step the catheter 26 is inserted. In this step the catheter 26 which is received in the cannula 12 is advanced through the cannula 12, e.g. with a piezo motor. Due to the guidance by the end portion 20 the catheter 26 exits the opening 22 in the insertion direction I such that the leading end of the catheter 26 moves through the intersection of directions I and P which coincides with the target location 46. Accordingly, at the target location 46 the catheter 26 penetrates the vessel 44.

In some embodiments the cannula 12 and the handle portion 14 are detachably connected, such that the cannula 12 may be disposable while the same handle portion 14 may be used again in further applications. Hence, in every application the instrument 10 may be used with a new cannula 12 to ensure that the cannula 12 is clean, flawless and/or sterile. Likewise to the cannula 12, also the catheter 26 may be disposable. Accordingly, the cannula 12 and the catheter 26, which may contain one or more drugs suitable for a respective treatment, can be replaced in combination. In this case, the entire combination of the cannula 12 and the catheter 26 corresponds to a disposable good or consumable. This allows ensuring an improved treatment both in terms of time and in terms quality.

For the specific application of ophthalmic surgery, the cannula 12 may have the following dimensions to which, however, the present invention is not restricted. The portion 36 of the first channel may have a diameter D₁ (indicated in Fig. 5) in a range of between 30 µm and 80 µm, for example of about 50 µm. The portion 38 of the first channel may have a diameter D₂ (indicated in Fig. 5) in a range of between 70 µm and 200 µm, for example of about 100 µm. The cannula 12 may have a length L₁ (indicated in Fig. 1) of between 30 mm and 60 mm, for example of about 40 mm. The middle portion 18 may have a length L₃ (indicated in Fig. 2) of between 300 µm and 2000 µm, for example of about 600 µm. The end portion 20 may have a length L₄ (indicated in Fig. 2) of between 100 µm and 400 µm, for example of about 200 µm. Accordingly, the length of the rear portion 16 may have a length L₂ (indicated in Fig. 1) in the range of the length L₁ of the cannula 12, i.e. in the range of between 30 mm and 60 mm, for example of about 39 mm.

The rear portion 16 of the cannula 12 may have a diameter D₃ (indicated in Fig. 2) of between 400 µm and 700 µm, for example of about 560 µm. The middle portion 18 of the cannula 12 may have a diameter D₄ (indicated in Fig. 2) of between 100 µm and 400 µm, for example of about 200 µm. The end portion 20 of the cannula 12 may have a diameter D₅ (indicated in Fig. 2) of between 60 µm and 150 µm, for example of about 80 µm.

The second channel 32 may have a diameter D₆ (indicated in Fig. 6) of between 70 µm and 200 µm, for example of about 100 µm.

The pointing beam 48 may have a diameter D₇ (indicated in Fig. 10b) which is preferably smaller than 200 µm and/or which at the intersection with the insertion direction I has a diameter which is smaller than 100 µm and more preferably about 50 µm or smaller. Although it may be preferred for some examples that the pointing beam 48 is collimated, the present invention is also related to applications where the pointing beam 48 has a certain divergence or is focused. Preferably the pointing beam 48 can create a spot on a ground at the intersection of the insertion direction and the pointing direction which is sufficiently defined and concentrated for the specific application. For the specific application of ophthalmic surgeries a spot size or beam diameter of 100 µm at the intersection of the insertion direction and the pointing direction can still be sufficient.

In preferred embodiments the pointing beam 48 has a diameter D₇ which essentially corresponds to the diameter of the catheter 26. For the application of ophthalmic surgeries the pointing beam 48 may have a diameter D₇ between 50 µm and 80 µm.

If the pointing beam 48 is not collimated, the spot size may serve as a measure for the distance of the cannula 12 from the ground.

While the present invention has been described with respect to specific examples and embodiments, it needs to be considered that the specific examples and embodiments are merely illustrative for the present invention and are not intended to limit the scope of the present invention which is defined by the appended claims.

### List of Reference Numerals

- 10: instrument
- 12: cannula
- 14: handle portion
- 16: rear portion of the cannula
- 18: middle portion of the cannula
- 20: end portion of the cannula
- 22: first opening
- 24: second opening
- 26: catheter
- 28: support element
- 30: first channel
- 32: second channel
- 34: end portion of the second channel
- 36: portion of first channel with smaller diameter
- 38: portion of first channel with larger diameter
- 40: eye ball
- 42: retina
- 44: retinal vessel
- 46: target location
- 48: pointing beam

## Claims

1. Cannula (12) for inserting a catheter (26), the cannula comprising a first channel (30) for receiving and guiding the catheter (26), the first channel extending to a first opening (22) and having an end portion defining an insertion direction (I), such that the catheter when being advanced and guided through the end portion of the first channel (30) exits the cannula (12) through the first opening (22) in the insertion direction (I), and
a second channel (32) for receiving a pointing device for providing a pointing beam (48), the second channel extending to a second opening (24) and having an end portion (34) defining a pointing direction (P), the pointing direction pointing from the second opening (32) towards a location, which is located outside the cannula (12) along the insertion direction (I) in front of the first opening (22).

2. Cannula (12) of claim 1, wherein the second channel (32) extends parallel to the first channel (30) and/or wherein the first channel (30) protrudes a leading end of the second channel (32).

3. Cannula (12) of claim 1 or 2, which has an end portion (20) located at the first opening (22) which is bent.

4. Cannula (12) of one of the preceding claims, wherein the first channel (30) and the second channel (32) are formed in the same single part of material.

5. Cannula (12) of one of the preceding claims, wherein the cannula (12) is a blunt cannula.

6. Cannula (12) of one of the preceding claims, further comprising a support element (28) being attached to an end portion (20) of the cannula for stabilizing the cannula when the cannula is forced against a ground (42, 44), wherein the supporting element preferably comprises a material which is softer than the material in which the first and second channels (30, 32) are defined.

7. Cannula (12) of one of the preceding claims, wherein the first channel (30) comprises a portion (38) having a first diameter (D₂) which is larger than a second diameter (D₁) of the end portion of the first channel (30),
wherein the first diameter (D₂) is preferably between 70 µm and 200 µm, in particular about 100 µm, and/or
wherein the second diameter (D₁) is preferably between 30 µm and 80 µm, in particular about 50 µm.

8. Cannula (12) of one of the preceding claims, wherein the cannula has a length (L₁) of between 30 mm and 60 mm, preferably of about 40 mm, and/or
a rear portion (16) having a diameter (D₃) of between 400 µm and 700 µm, preferably of about 560 µm, and/or
a middle portion (18) having a diameter (D₄) of between 100 µm and 400 µm, preferably of about 200 µm, and/or
an end portion (20) having a diameter (D₅) of between 60 µm and 150 µm, preferably of about 80 µm.

9. Instrument (10) comprising
a cannula (12) of one of the preceding claims and
a pointing device being received in the second channel (32).

10. Instrument for inserting a catheter (26), the instrument comprising a cannula having a channel (30) for receiving and guiding the catheter (26), the channel (30) extending to an opening (22) and having an end portion defining an insertion direction (I), such that the catheter (26) when being advanced and guided through the end portion of the channel (30) exits the cannula through the opening (22) in the insertion direction (I), and
a pointing device for providing a pointing beam (48) in a pointing direction (P), wherein the pointing device is attached to the cannula and relatively arranged to the cannula, such that - when a pointing beam (48) is provided - the pointing beam points and propagates towards a location, which is located outside the cannula along the insertion direction (I) in front of the opening (22).

11. Instrument (10) of claim 9 or 10, wherein the pointing device comprises an optical fiber and/or a lens.

12. Instrument (10) of one of claims 9 to 11, wherein the pointing device further comprises a light source.

13. Instrument (10) of one of claims 9 to 12, further comprising a handle portion (14),
wherein the handle portion preferably comprises
a mechanism for moving a catheter (26) and/or
a mechanism for fluid injection and/or fluid removal, and/or
wherein the handle portion (14) and the cannula (12) are detachably connectable, wherein the cannula (12) is preferably disposable.

14. Instrument (10) of one of claims 9 to 13 or instrument comprising a cannula of one of claims 1 to 8, further comprising a catheter (26) being receivable in the cannula (12), wherein the catheter preferably has
a leading end which is pointed and/or
a portion having a larger diameter than a leading portion of the catheter (26).

15. Instrument (10) of one of claims 9 to 14, which is configured to provide the pointing beam (48) inside an eye ball (40), preferably inside a human eye ball.
